# EUROPÄISCHE PATENTANMELDUNG

(11) **EP 3 492 117 A1**
(43) Veröffentlichungstag der Anmeldung: **05.06.2019**
(21) Anmeldenummer: 17204889.4
(22) Anmeldetag: 01.12.2017
(51) Int. Cl.: A61M 1/10, A61M 1/12

(54) **BLUTPUMPE**

(71) Anmelder: Berlin Heart GmbH, 12247 Berlin (DE)
(72) Erfinder: WINTERWERBER, Kim Peter, 12357 Berlin (DE); SCHMIDT, Bodo, 14513 Teltow (DE); WISNIEWSKI, Adrian, 10963 Berlin (DE)
(74) Vertreter: Pfenning, Meinig & Partner mbB

(57) **Zusammenfassung**

Die Anmeldung betrifft eine Blutpumpe, die ein Gehäuse (1) mit einem Einlass (2) und einem Auslass (3) und einen Rotor (4) zur Förderung von Fluid vom Einlass (2) zum Auslass (3) aufweist. Der Rotor (4) umfasst einen Zentralkörper (41), über welchen der Rotor (4) um eine Rotationsachse (5) des Rotors (4) drehbar gelagert ist, einen ersten Ringkörper(42), welcher auf einer Einlassseite des Rotors (4) angeordnet und einstückig mit dem Zentralkörper (41) verbunden ist, und Förderelemente (43), welche sich auf einer Auslassseite des erstens Ringkörpers (42) und im Wesentlichen senkrecht zur Rotationsachse (5) erstrecken und einstückig direkt mit dem ersten Ringkörper (42) und einstückig direkt mit dem Zentralkörper (41) verbunden sind.

## Beschreibung

Die vorliegende Erfindung betrifft eine Blutpumpe.

Eine wichtige Eigenschaft moderner Blutpumpen betrifft den Grad der Blutschädigung. Je nach Pumpenkonzept und insbesondere Lagerkonzept kann das durch die Pumpe strömende Blut unterschiedlich stark geschädigt werden. Angestrebt wird eine möglichst geringe Blutschädigung, etwa durch möglichst strömungsgünstige Formen, große Spaltmaße etc. Insbesondere können jedoch rotierende Streben des Rotors, welche ein zentrales Rotorelement mit den Schaufeln zur Förderung des Blutes verbinden, eine Blutschädigung bewirken, da das Blut beim Durchströmen durch die Pumpe die rotierenden Streben umspült.

Bei den im Stand der Technik bekannten Halbaxial- oder Radialpumpen ist der Rotor im Allgemeinen aus einer großen Anzahl separater Teile aufgebaut. Dies verkompliziert die Herstellung der Blutpumpen, da die einzelnen Bestandteile präzise zueinander passend gefertigt und montiert werden müssen. Des Weiteren können die innerhalb der Pumpe wirkenden Kräfte auf separate Bestandteile unterschiedlich stark einwirken, was die Pumpleistung negativ beeinflusst. Insbesondere ist die Montage der separaten Streben, welche das zentrale Rotorelement mit den Schaufeln verbinden, bei der Herstellung der Blutpumpen aufwändig.

Die im Stand der Technik bekannten Halbaxial- oder Radialpumpen haben daher den Nachteil, dass sowohl die Herstellung der Blutpumpe aufwändig ist, als auch dass die Blutschädigung ungünstig beeinflusst wird.

Die Aufgabe der vorliegenden Erfindung ist es daher, eine Blutpumpe bereitzustellen, die einerseits einfacher zu fertigen ist und anderseits eine geringere Blutschädigung mit sich bringt.

Die Aufgabe wird durch eine Blutpumpe gemäß Anspruch 1 gelöst. Vorteilhafte Weiterbildungen der erfindungsgemäßen Blutpumpe sind in den abhängigen Ansprüchen aufgeführt.

Die erfindungsgemäße Blutpumpe, umfasst ein Gehäuse mit einem Einlass und einem Auslass sowie einen Rotor zur Förderung von Fluid, insbesondere von Blut, vom Einlass zum Auslass. Der Rotor weist einen Zentralkörper auf, über welchen der Rotor um eine Rotationsachse des Rotors drehbar gelagert ist. Weiterhin weist der Rotor einen ersten Ringkörper (im Folgenden auch mit Deckscheibe bezeichnet) auf, welcher auf einer Einlassseite des Rotors angeordnet und einstückig mit dem Zentralkörper verbunden ist. Der Rotor weist weiterhin Förderelemente auf, welche sich auf einer Auslassseite des erstens Ringkörpers und im Wesentlichen senkrecht zur Rotationsachse erstrecken und einstückig direkt mit dem ersten Ringkörper und einstückig direkt mit dem Zentralkörper verbunden sind.

In der Beschreibung und in den Ansprüchen wird eine dem Einlass zugewandte Seite in Bezug auf eine Richtung der Rotationsachse (axiale Richtung) oder in Bezug auf eine Strömungsrichtung als Einlassseite oder einlassseitig bezeichnet. Als Auslassseite bzw. auslassseitig wird eine der Einlassseite gegenüberliegende Seite in Bezug auf die Richtung der Rotationsachse des Rotors und/oder in Bezug auf die Strömungsrichtung des Fluids verstanden.

Die erfindungsgemäße Blutpumpe weist somit einen Rotor auf, bei dem der Zentralkörper, der erste Ringkörper und die Förderelemente keine separaten Bestandteile sind, die präzise auf einander abgestimmt gefertigt und separat in dem Gehäuse der Blutpumpe montiert werden müssen. Außerdem wird eine bessere Pumpleistung erreicht, da sich beim Betrieb der Pumpe sämtliche Bestandteile des Rotors wie ein einstückiges Bauteil verhalten, welches einheitlichen Kräften innerhalb der Pumpe ausgesetzt ist. Durch die spezielle Form des Rotors, bei der die Förderelemente direkt am Zentralkörper ansetzen und den ersten Ringkörper mit dem Zentralkörper verbinden, kann die Blutschädigung verringert werden, da auf die im Stand der Technik bekannten rotierenden Streben verzichtet und das Blut, ohne ein weiteres rotierendes Hindernis passieren zu müssen, direkt in die Förderelemente eingeleitet werden kann.

In einer besonders bevorzugten Ausgestaltung der Erfindung sind der Zentralkörper, der erste Ringkörper und/oder die Förderelemente einstückig, insbesondere mittels Fräsens, gefertigt. Ferner ist es denkbar, dass der Zentralkörper, der erste Ringkörper und/oder die Förderelemente stoffschlüssig miteinander verbunden, insbesondere verschweißt.

Der Rotor kann frei drehbar axial und/oder radial mechanisch gelagert sein. Für den Fall, dass der Rotor frei drehbar magnetisch gelagert ist, kann das magnetische Lager ein Radial-, Axial- und/oder Kipplager sein. Eine magnetische Lagerung ist insbesondere in Bezug auf die Blutschädigung vorteilhaft.

Der Rotor ist vorzugsweise derart im Gehäuse angeordnet, dass ein Abstand zwischen den Förderelementen und dem sich auslassseitig des Rotors befindlichen Boden des Gehäuses minimal ist. Dadurch kann das zu fördernde Blut optimal zwischen den Förderelementen, also in einer Ebene senkrecht zur Rotationsachse, geführt und zum Auslass transportiert werden.

Weiterhin kann der Rotor auslassseitig einen zweiten Ringkörper (im Folgenden auch mit Tragscheibe bezeichnet) zur Führung des Fluids in der Ebene senkrecht zur Rotationsachse durch die Förderelemente aufweisen, wobei der zweite Ringkörper insbesondere einstückig mit den Förderelementen verbunden ist. Durch die einstückige Verbindung zwischen dem zweiten Ringkörper und den Förderelementen bzw. dem übrigen Rotor kann sich bei einem Ansaugen des zweiten Ringkörpers am Boden des Gehäuses, beispielsweise aufgrund einer Sekundärströmung des Blutes, ein Abstand zwischen dem zweiten Ringkörper und den Förderelementen nicht vergrößern. Dies gewährleistet eine sichere Führung des Blutes durch die Förderelemente und somit eine gute Pumpleistung.

Ein weiterer Vorteil eines Rotors mit zweitem Ringkörper besteht darin, dass ein axiales Druck- und Kraftgleichgewicht leichter einstellbar ist. Durch die ähnliche Strömungsführung auf der Einlassseite und der Auslassseite des Rotors heben sich die resultierenden Kräfte nahezu auf.

Besonders vorteilhaft ist es, wenn der zweite Ringkörper einstückig mit den Förderelementen gefertigt, insbesondere mittels Fräsens, oder stoffschlüssig mit den Förderelementen verbunden, insbesondere verschweißt, sind. Der zweite Ringkörper kann auch über eine Stiftverbindung mit dem ersten Ringkörper, den Förderelementen und/oder dem Zentralkörper verbunden sein.

In einer weiteren vorteilhaften Ausgestaltung der Erfindung können der erste und/oder der zweite Ringkörper einen Rotormagneten oder mehrere Rotormagneten zum Antrieb des Rotors aufweisen.

Weist dagegen der Rotor keinen zweiten Ringkörper auf, kann sich eine resultierende axiale Kraft auf den Rotor ergeben, welche einen positiven Effekt auf die Lagerstabilität und/oder die Strömungsanalyse des Rotors hat.

Weiterhin kann in oder an dem Gehäuse benachbart zum ersten Ringkörper eine Motorspule angeordnet sein, sodass der Rotor mittels einer magnetischen Wechselwirkung zwischen dem Rotormagneten und der Motorspule antreibbar ist.

Die Motorspule kann einlassseitig und/oder auslassseitig des Rotors angeordnet sein.

Der Rotormagnet und die Motorspule können symmetrisch, rotationssymmetrisch oder unsymmetrisch um die Rotationsachse des Rotors ausgebildet sein.

Im ersten und/oder zweiten Ringkörper kann ein erster Lagermagnet und im oder am Gehäuse ein zweiter Lagermagnet angeordnet sein, welche derart ausgebildet sind, dass der Rotor durch eine Wechselwirkung des ersten und zweiten Lagermagneten im Wesentlichen in einer Rotationsebene des Rotors gehalten wird.

Der erste und der zweite Lagermagnet können in axialer Richtung versetzt zueinander angeordnet sein, sodass der Rotor durch eine Wechselwirkung des ersten und zweiten Lagermagneten in Richtung des Auslasses vorgespannt gehalten wird.

Der erste und/oder der zweite Lagermagnet können symmetrisch, rotationssymmetrisch oder unsymmetrisch um die Rotationsachse des Rotors ausgebildet sein.

Eine Wandung des Gehäuses kann auslassseitig des Zentralkörpers und im Bereich der Rotationsachse nach innen vorspringen und eine Fassung, insbesondere eine nahezu halbkugelförmige Fassung, oder eine Kalotte aufweisen, und der Zentralkörper auslassseitig entsprechend eine Kalotte oder eine Fassung, insbesondere eine nahezu halbkugelförmige Fassung, aufweisen, wobei die Kalotte in der Fassung axial und/oder radial gelagert ist.

Weiterhin ist es denkbar, dass einlassseitig des Rotors ein mechanisches Lager angeordnet ist, welches mittels Streben mit dem Gehäuse verbunden ist und in welchem der Zentralkörper einlassseitig axial und/oder radial gelagert ist.

Der Einlass der Blutpumpe kann axial ausgerichtet sein, sodass das Fluid im Wesentlichen entlang der Rotationsachse des Rotors in die Blutpumpe einführbar ist.

Der Auslass der Blutpumpe kann radial und/oder tangential ausgerichtet in einer Wandung des Gehäuses angeordnet sein, sodass das Fluid im Wesentlichen in radialer und/oder tangentialer Richtung aus der Blutpumpe abführbar ist.

Des Weiteren könnender erste und/oder der zweite Ringkörper benachbart zur Rotationsachse eine oder mehrere parallel zur Rotationsachse verlaufende Durchgangsöffnungen zum Zuleiten des Fluids zu den Förderelementen aufweisen.

Im Folgenden wird eine erfindungsgemäße Blutpumpe anhand von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des jeweiligen Beispiels und weiterer Merkmale des jeweiligen Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

Es zeigen
- Figur 1: eine schematische Darstellung eines ersten Ausführungsbeispiels einer erfindungsgemäßen Blutpumpe in einer Schnittansicht parallel zur Rotationsachse,
- Figur 2: eine schematische Darstellung eines zweiten Ausführungsbeispiels einer erfindungsgemäßen Blutpumpe in einer Schnittansicht parallel zur Rotationsachse,
- Figur 3: eine schematische Darstellung eines dritten Ausführungsbeispiels einer erfindungsgemäßen Blutpumpe in einer Schnittansicht parallel zur Rotationsachse und
- Figur 4: eine schematische Darstellung eines vierten Ausführungsbeispiels einer erfindungsgemäßen Blutpumpe in einer Schnittansicht parallel zur Rotationsachse.

In den Figuren werden die folgenden Bezugszeichen verwendet:
- B: Blut
- 1: Gehäuse
- 11: Gehäuseboden
- 11a: Vorsprung
- 11b: hohlzylinderförmige Wandung
- 2: Einlass
- 3: Auslass
- 4: Rotor
- 41: Zentralkörper
- 42: erster Ringkörper (Deckscheibe)
- 43: Förderelemente
- 44: zweiter Ringkörper (Tragscheibe)
- 5: Rotationsachse
- 6: mechanisches Lager
- 61: Kalotte
- 62: halbkugelförmige Fassung
- 63: Steg
- 7: Rotormagnet
- 8: Motorspule
- 9: erster Lagermagnet
- 10: zweiter Lagermagnet
- 12: Eisenkern

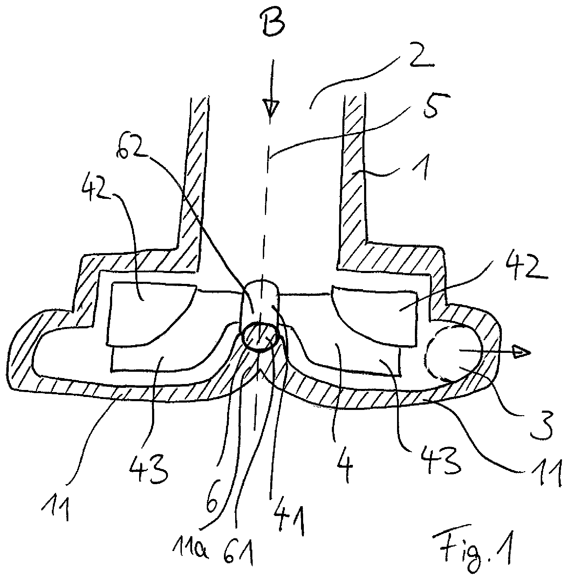

Figur 1 zeigt ein erstes Ausführungsbeispiel einer erfindungsgemäßen Blutpumpe in einer Schnittansicht parallel zu einer Rotationsachse 5. Die Blutpumpe weist ein Gehäuse 1 mit einem Einlass 2 und einem Auslass 3 auf. Innerhalb des Gehäuses 1 ist ferner ein Rotor 4 zur Förderung von Fluid (Blut B) vom Einlass 2 zum Auslass 3. Der Einlass 2 ist derart am Gehäuse 1 angeordnet, dass das Blut B entlang der Rotationsachse 5 des Rotors 4 in die Blutpumpe eintritt. Der Auslass 3 ist auf einer dem Einlass 2 gegenüberliegenden Seite der Blutpumpe senkrecht zur Rotationsachse 5 versetzt angeordnet. Der Auslass 3 kann dabei so ausgerichtet sein, dass das Blut B in einer radialen Richtung oder einer tangentialen Richtung aus dem Gehäuse 1 austritt.

Zur weiteren Beschreibung der Figuren wird eine dem Einlass 2 zugewandte Seite in Bezug auf die Richtung der Rotationsachse 5 (axiale Richtung) als Einlassseite oder einlassseitig bezeichnet. Weiterhin wird eine dem Einlass 2 abgewandte Seite in Bezug auf die Richtung der Rotationsachse 5 als Auslassseite bzw. auslassseitig bezeichnet.

Der Rotor 4 ist in der Mitte des Gehäuses 1 angeordnet. Der Rotor 4 weist einen Zentralkörper 41 auf, welcher auf einem mechanischen Lager 6 gelagert ist. Weiterhin weist der Rotor 4 einlassseitig einen ersten Ringkörper 42 auf, der ringförmig ausgebildet ist und rotationssymmetrisch um die Rotationsachse 5 angeordnet ist. Im Bereich der Rotationsachse 5 weist der erste Ringkörper 42 eine kreisförmige Aussparung auf, in welche der Zentralkörper 41 hineinragt. Weiterhin weist der Rotor 4 schaufelartige Förderelemente 43 auf, welche sich auf einer Auslassseite des ersten Ringkörpers 42 im Wesentlichen senkrecht zur Rotationsachse 5 erstrecken. Die Förderelemente 43 sind in axialer Richtung einstückig direkt mit dem ersten Ringkörper 42 verbunden. Zur Rotationsachse 5 hin sind die Förderelemente 43 in Richtung des Einlasses 2 bogenförmig in die Aussparung des ersten Ringkörpers 42 hinein und an den Zentralkörper 41 heran geführt und senkrecht zur axialen Richtung auf einer von der Rotationsachse 5 abgewandten Seite einstückig direkt mit dem Ringkörper 42 und auf einer der Rotationsachse 5 zugewandten Seite einstückig direkt mit dem Zentralkörper 41 verbunden.

Der auslassseitig gelegene Gehäuseboden 11 des Gehäuses 1 verläuft entsprechend der auslassseitigen Form des Rotors 4. In einem von der Rotationsachse 5 beabstandeten Bereich ist der Gehäuseboden 11 im Wesentlichen plan und verläuft in etwa senkrecht zur Rotationsachse 5. Im zentralen Bereich, also im Bereich der Rotationsachse 5 springt der Gehäuseboden 11 nach innen, entsprechend dem Verlauf der Förderelemente 43, bogenförmig vor, sodass auslassseitig zwischen den Förderelementen 43 und dem Gehäuseboden 11 lediglich ein minimaler Abstand verbleibt. Der Abstand ist so gewählt, dass eine Rotation des Rotors 4 ermöglicht wird, jedoch kein Teil des Blutes B an den schaufelartigen Förderelementen 43 vorbei laufen kann.

Das Lager 6 ist auf dem auf der Rotationsachse 5 liegenden Vorsprung 11a des Gehäusebodens 11 angeordnet. Das Lager 6 umfasst eine auf dem Vorsprung 11a befestigte, feststehende Kalotte 61, die mit einer auslassseitig ausgebildeten, halbkugelförmigen Fassung 62 des Zentralkörpers 41 zusammenwirkt, sodass der Zentralkörper 41 bei der Rotation des Rotors 4 auf der Kalotte 61 gleitet. Mittels des Lagers 6 ist der Rotor 4 radial und axial in seiner Position festgelegt.

Bei dem vorstehend beschriebenen Ausführungsbeispiel einer erfindungsgemäßen Blutpumpe ist ersichtlich, dass durch die spezielle Form der Förderelemente 43, welche bis an den Zentralkörper 41 herangeführt sind, auf zusätzliche, mitrotierende Streben zur Verbindung des Zentralkörpers 41 mit dem ersten Ringkörper 42 verzichtet werden kann. Bei dem vorliegenden Ausführungsbeispiel wird das in den Rotor 4 eintretende Blut B direkt den Förderelementen 43 zugeführt. Hierdurch wird eine Blutschädigung minimiert, da das Blut vor dem Zuführen zu den Förderelementen nicht noch rotierende Streben passieren muss. Des Weiteren ist eine Fertigung der vorstehend beschriebenen Blutpumpe vereinfacht, da der Rotor 4 eine geringe Anzahl von einstückig miteinander verbundenen Bestandteilen aufweist. Insbesondere kann auf eine Montage von zusätzlichen Streben zur Verbindung des ersten Ringkörpers 42 mit dem Zentralkörper 41 verzichtet werden. Ein zusätzlicher Vorteil des ersten Ausführungsbeispiels besteht darin, dass aufgrund des geringen Abstands zwischen dem Gehäuseboden 11 und der Auslassseite der Förderelemente 43 auf eine Tragscheibe (zweiter Ringkörper), welche bei im Stand der Technik bekannten Blutpumpen ein auslassseitiges Vorbeilaufen des Blutes an den Förderelementen 43 verhindert, verzichtet werden kann.

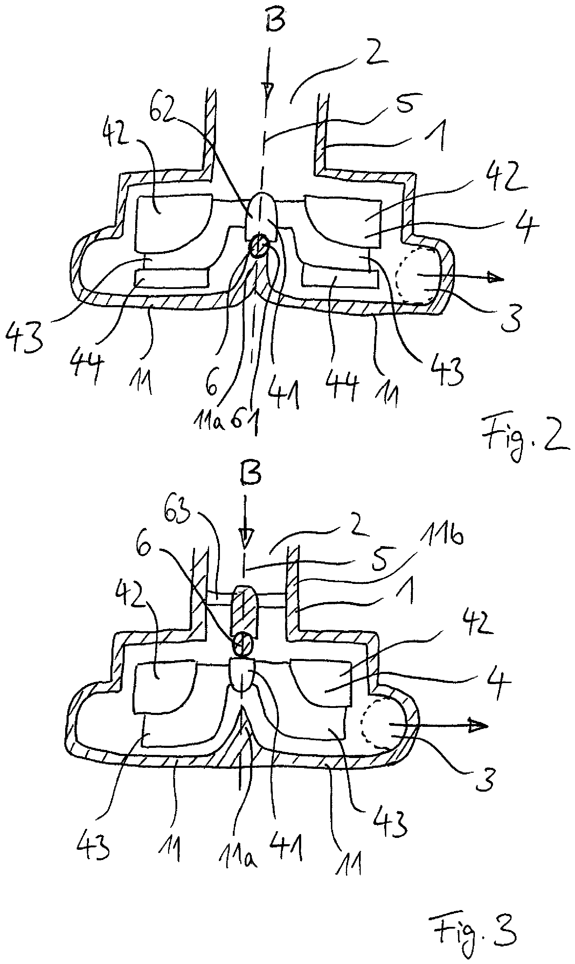

Figur 2 zeigt eine schematische Darstellung eines zweiten Ausführungsbeispiels einer erfindungsgemäßen Blutpumpe in einer Schnittansicht parallel zur Rotationsachse 5. Die Blutpumpe nach dem zweiten Ausführungsbeispiel in Figur 2 weist im Wesentlichen den gleichen Aufbau auf wie die Blutpumpe aus Figur 1. Im Vergleich zur Blutpumpe der Figur 1 ist der auslassseitige Abstand zwischen den Förderelementen 43 und dem Gehäuseboden 11 vergrößert. Weiterhin ist auslassseitig an den Förderelementen 43 ein zweiter Ringkörper 44 angeordnet und einstückig mit den Förderelementen 43 verbunden. Im zentralen Bereich, also im Bereich der Rotationsachse 5 weist der zweite Ringkörper 44 eine Aussparung auf, durch welche der Vorsprung 11a hindurchtritt. Der zweite Ringkörper 44 schließt den Rotor 4 auslassseitig ab und dient der Führung des Blutes B senkrecht zur Rotationsachse 5. Der zweite Ringkörper 44 verhindert somit, dass Blut B auslassseitig an den Förderelementen 43 vorbei laufen kann.

Die Blutpumpe gemäß dem zweiten Ausführungsbeispiel weist ebenfalls die Vorteile der geringeren Blutschädigung und der vereinfachten Fertigung auf. Ein zusätzlicher Vorteil der Blutpumpe der Figur 2 besteht darin, dass ein oder mehrere Rotormagneten zum Antrieb des Rotors 4 und ein oder mehrere Lagermagneten zur axialen Vorspannung des Rotors 4 entweder sowohl im ersten 42 und im zweiten Ringkörper 44 angeordnet werden können oder, alternativ zu einer Anordnung im ersten Ringkörper 42, im zweiten Ringkörper 44 angeordnet werden können.

Figur 3 zeigt eine schematische Darstellung eines dritten Ausführungsbeispiels einer erfindungsgemäßen Blutpumpe in einer Schnittansicht parallel zur Rotationsachse 5. Die Blutpumpe nach dem dritten Ausführungsbeispiel in Figur 3 weist einen ähnlichen Aufbau auf wie die Blutpumpe aus Figur 1 auf. Der einzige Unterschied zur Blutpumpe in Figur 1 besteht dabei in einer alternativen mechanischen Lagerung des Rotors 4. Beim dritten Ausführungsbeispiel ist der Rotor 4 über den Zentralkörper 41 an dem Lager 6 aufgehängt. Das Lager 6 ist mittels zwei oder mehreren Stegen 63 an einer hohlzylinderförmigen Wandung 11b des Gehäuses 1 im Bereich des Einlasses 2 befestigt. Die Stege 63 verlaufen dabei radial von dem sich auf der Rotationsachse 5 befindlichen Lager 6 zur Wandung 11b. Der Rotor 4 ist in Figur 3 somit einlassseitig mit dem Gehäuse 1 verbunden und hängt auslassseitig frei. Durch das Lager 6 ist der Rotor 4 radial und axial festgelegt.

Die Blutpumpe gemäß dem dritten Ausführungsbeispiel weist ebenfalls die Vorteile der Blutpumpe gemäß dem ersten Ausführungsbeispiel auf.

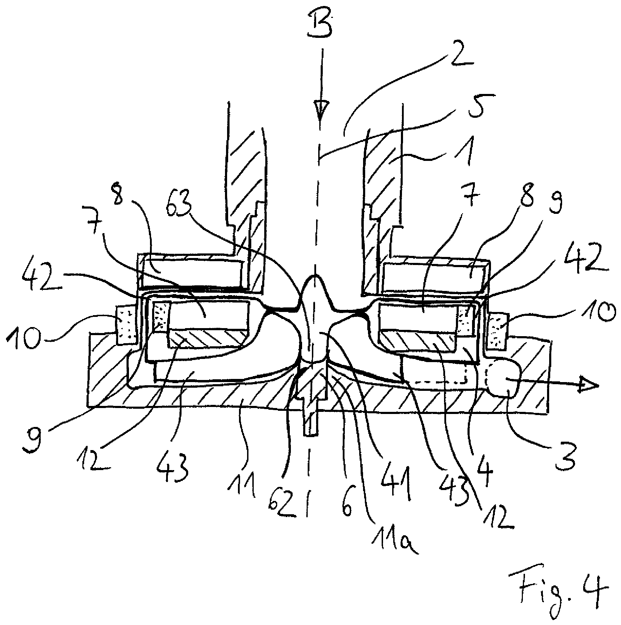

Figur 4 zeigt eine schematische Darstellung eines vierten Ausführungsbeispiels einer erfindungsgemäßen Blutpumpe in einer Schnittansicht parallel zur Rotationsachse 5. Das vierte Ausführungsbeispiel entspricht vom Aufbau her dem ersten Ausführungsbeispiel in Figur 1. Eine vollständige Beschreibung des Aufbaus der Blutpumpe in Figur 4 kann hier daher weggelassen werden. Im Vergleich zur Figur 1 sind in Figur 4 zusätzlich ein Rotormagnet 7, eine Motorspule 8, ein erster Lagermagnet 9, ein zweiter Lagermagnet 10 und ein Eisenkern 12 dargestellt.

Der Rotormagnet 7 ist ringförmig ausgebildet und auf einer Einlassseite im ersten Ringkörper 42 angeordnet. Entsprechend ist einlassseitig des ersten Ringkörpers 42 im Gehäuse 1 oder auf dem Gehäuse 1 eine ringförmige Motorspule 8 angeordnet. Die Motorspule 8 wirkt mit dem Rotormagneten 7 zusammen, um den Rotor 4 anzutreiben. Zur Verstärkung des Magnetflusses zwischen der Motorspule 8 und dem Rotormagneten 7 ist ebenfalls im ersten Ringkörper 42 auslassseitig des Rotormagneten 7 ein ringförmiger Eisenkern 12 angeordnet.

Des Weiteren ist auf einer der Rotationsachse 5 abgewandten Seite des Rotormagneten 7 im ersten Ringkörper 42 ein erster ringförmiger Lagermagnet 9 angeordnet. Auf einer der Rotationsachse 5 abgewandten Seite des ersten Lagermagneten 9 ist im oder am Gehäuse 1 ein zweiter ringförmiger Lagermagnet 10 angeordnet, der axial zur Auslassseite hin versetzt zum ersten Lagermagneten 9 ist, jedoch noch mit dem ersten Lagermagneten 9 in axialer Richtung überlappt. Der erste und zweite Lagermagnet 9 und 10 wirken zusammen, um den Rotor 4 gegenüber Kippelbewegungen zu stabilisieren und den Rotor 4 zur Auslassseite hin vorzuspannen. Durch die magnetische Vorspannung in Richtung der Auslassseite kann ein Axialschub des Rotors 4 bei einer fluidfördernden Rotation des Rotors 4 kompensiert werden.

Ein weiterer Unterschied zur Blutpumpe des ersten Ausführungsbeispiels besteht in der Ausgestaltung des Lagers 6. Bei der Blutpumpe in Figur 4 weist der Zentralkörper 41 auslassseitig eine Kalotte 63 auf, welche in einer halbkugelförmigen Fassung 62 des Lagers 6 gelagert ist. Durch das Lager 6 ist der Rotor 4 radial und axial festgelegt.

Die Blutpumpe gemäß dem vierten Ausführungsbeispiel weist zudem die Vorteile der Blutpumpe gemäß dem ersten Ausführungsbeispiel auf.

## Patentansprüche

1. Blutpumpe, umfassend
ein Gehäuse mit einem Einlass und einem Auslass,
einen Rotor zur Förderung von Fluid vom Einlass zum Auslass, wobei der Rotor
einen Zentralkörper, über welchen der Rotor um eine Rotationsachse des Rotors drehbar gelagert ist,
einen ersten Ringkörper, welcher auf einer Einlassseite des Rotors angeordnet und einstückig mit dem Zentralkörper verbunden ist,
und Förderelemente, welche sich auf einer Auslassseite des erstens Ringkörpers und im Wesentlichen senkrecht zur Rotationsachse erstrecken und einstückig direkt mit dem ersten Ringkörper und einstückig direkt mit dem Zentralkörper verbunden sind, aufweist.

2. Blutpumpe nach dem vorhergehenden Anspruch, wobei der Rotor frei drehbar axial und/oder radial mechanisch gelagert ist.

3. Blutpumpe nach einem der vorhergehenden Ansprüche, wobei der Rotor frei drehbar und teilweise oder vollständig magnetisch gelagert ist, und das magnetische Lager ein Radial-, Axial- und/oder Kipplager ist.

4. Blutpumpe nach einem der vorhergehenden Ansprüche, wobei der Rotor auslassseitig einen zweiten Ringkörper zur Führung des Fluids in der Ebene senkrecht zur Rotationsachse durch die Förderelemente aufweist, wobei der zweite Ringkörper insbesondere einstückig mit den Förderelementen verbunden ist.

5. Blutpumpe nach einem der vorhergehenden Ansprüche, wobei der erste und/oder der zweite Ringkörper einen Rotormagneten zum Antrieb des Rotors aufweisen.

6. Blutpumpe nach dem vorhergehenden Anspruch, wobei in oder an dem Gehäuse benachbart zum ersten Ringkörper eine Motorspule angeordnet ist, sodass der Rotor mittels einer magnetischen Wechselwirkung zwischen dem Rotormagneten und der Motorspule antreibbar ist.

7. Blutpumpe nach dem vorhergehenden Anspruch, wobei die Motorspule einlassseitig und/oder auslassseitig des Rotors angeordnet ist.

8. Blutpumpe nach einem der drei vorhergehenden Ansprüche, wobei der Rotormagnet und die Motorspule rotationssymmetrisch um die Rotationsachse des Rotors ausgebildet sind.

9. Blutpumpe nach einem der vorhergehenden Ansprüche, wobei im ersten und/oder zweiten Ringkörper ein erster Lagermagnet und im oder am Gehäuse ein zweiter Lagermagnet angeordnet sind, welche derart ausgebildet sind, dass der Rotor durch eine Wechselwirkung des ersten und zweiten Lagermagneten im Wesentlichen in einer Rotationsebene des Rotors gehalten wird.

10. Blutpumpe nach dem vorhergehenden Anspruch, wobei der erste und der zweite Lagermagnet in axialer Richtung versetzt zueinander angeordnet sind, sodass der Rotor durch eine Wechselwirkung des ersten und zweiten Lagermagneten in Richtung des Auslasses vorgespannt gehalten wird.

11. Blutpumpe nach einem der zwei vorhergehenden Ansprüche, wobei der erste und/oder der zweite Lagermagnet rotationssymmetrisch um die Rotationsachse des Rotors ausgebildet sind.

12. Blutpumpe nach einem der vorhergehenden Ansprüche, wobei eine Wandung des Gehäuses auslassseitig des Zentralkörpers und im Bereich der Rotationsachse nach innen vorspringt und eine halbkugelförmige Fassung oder eine Kalotte aufweist, und der Zentralkörper auslassseitig entsprechend eine Kalotte oder eine halbkugelförmige Fassung aufweist, wobei die Kalotte in der halbkugelförmigen Fassung axial und radial gelagert ist.

13. Blutpumpe nach einem der Ansprüche 1 bis 11, wobei einlassseitig des Rotors ein mechanisches Lager angeordnet ist, welches mittels Streben mit dem Gehäuse verbunden ist und in welchem der Zentralkörper einlassseitig axial und/oder radial gelagert ist.

14. Blutpumpe nach einem der vorhergehenden Ansprüche, wobei der Auslass radial und/oder tangential ausgerichtet in einer Wandung des Gehäuses angeordnet ist, sodass das Fluid im Wesentlichen in radialer und/oder tangentialer Richtung aus der Blutpumpe abführbar ist.

15. Blutpumpe nach einem der vorhergehenden Ansprüche, wobei der erste und/oder der zweite Ringkörper benachbart zur Rotationsachse eine oder mehrere parallel zur Rotationsachse verlaufende Durchgangsöffnungen zum Zuleiten des Fluids zu den Förderelementen aufweisen.
